(19) Europäisches Patentamt
European Patent Office
Office européen des brevets

(11) Numéro de publication : 0 206 958 B1

(12) # FASCICULE DE BREVET EUROPÉEN

(45) Date de publication du fascicule du brevet : 07.09.88

(51) Int. Cl.⁴ : C 07 C 69/76, C 07 C 67/26

(21) Numéro de dépôt : 86420159.5

(22) Date de dépôt : 20.06.86

(54) **Procédé de préparation d'acides carboxyliques et de leurs esters.**

(30) Priorité : 21.06.85 FR 8509675

(43) Date de publication de la demande : 30.12.86 Bulletin 86/52

(45) Mention de la délivrance du brevet : 07.09.88 Bulletin 88/36

(84) Etats contractants désignés : AT BE CH DE FR GB IT LI LU NL SE

(56) Documents cités : EP-A- 0 112 679

(73) Titulaire : RHONE-POULENC CHIMIE
25, quai Paul Doumer
F-92408 Courbevoie Cédex (FR)

(72) Inventeur : Basset, Jean-Marie
21, Petite rue de la Doua
F-69100 Villeurbanne (FR)
Inventeur : Mutin, Robert
21, rue Ernest Renan
F-69330 Meyzieu (FR)

(74) Mandataire : Le Pennec, Magali et al
RHONE-POULENC INTERSERVICES Service Brevets
Chimie 25, Quai Paul Doumer
F-92408 Courbevoie Cédex (FR)

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

**Description**

La présente invention a pour objet un procédé de préparation d'acides carboxyliques aromatiques et de leurs esters par hydroxy- ou alkoxycarbonylation de composés aromatiques halogénés par l'oxyde de carbone en présence d'eau et/ou d'un alcool.

La préparation d'acides carboxyliques aromatiques et de leurs esters par carbonylation (hydroxycarbonylation ou alkoxycarbonylation) des halogénures aromatiques présente un grand intérêt industriel en raison de l'abondance de l'oxyde de carbone et de son faible coût. C'est pour cette raison que la carbonylation des halogénures a fait l'objet de travaux de recherches ayant pour objectif la mise au point de catalyseurs ayant une productivité aussi élevée que possible dans des conditions de pression et de température relativement douces. Parmi les différents catalyseurs proposés les complexes du palladium avec des phosphines se sont révélés particulièrement intéressant de ce point de vue (cf. D. VALENTINE et al., J. Org. Chem., 46, 4614-4617 [1981] ; M. HIDAI et al., Bull. Chem. Soc. Japan, 48 (7), 2075-2077 [1975]). Cependant l'intérêt des complexes palladium/phosphines est limité par le fait qu'ils ne permettent pas la carbonylation des chlorures aromatiques. Leur utilisation est par conséquent circonscrite à la carbonylation de certains bromures et iodures aromatiques. Or l'industrie porte un grand intérêt à la carbonylation des composés chloroaromatiques, produits de départ courants moins coûteux que les bromures et les iodures. La présente invention a pour objet de résoudre le problème posé par l'activation de la liaison chlore/carbone d'un cycle aromatique de façon à rendre possible la carbonylation des chlorures aromatiques. Par ailleurs il s'avère que la carbonylation de bromures aromatiques comportant des substituants électrodonneurs (dialkylamino par exemple) est, elle aussi, difficile. Un autre objet de la présente invention réside par conséquent dans un procédé de carbonylation des bromures aromatiques comportant des substituants électrodonneurs.

Plus spécifiquement la présente invention a pour objet un procédé de préparation d'acides carboxyliques aromatiques et/ou de leurs esters par carbonylation de composés chloro- ou bromoaromatiques en présence d'eau et/ou d'un alcanol, d'un accepteur d'hydracide, d'un complexe palladium/phosphine et éventuellement d'une phosphine caractérisé en ce que l'on soumet à la carbonylation des chloro- ou bromoarènechromecarbonyles.

On a constaté de manière inattendue que la présence d'un reste chromecarbonyle —$Cr(CO)_3$ sur le noyau aromatique des chlorures aromatiques et des bromures aromatiques à substituants électrodonneurs rend possible la carbonylation de ces composés en présence de complexes palladium/phosphines.

Les composés arènechrometricarbonyles sont des produits connus qui peuvent être obtenus aisément par diverses voies de synthèse (cf. G. WILKINSON et al., Comprehensive Organometallic Chemistry, vol. 3, pages 1001-1021, édité par Pergamon Press). Une voie d'accès préférée aux arènechrometricarbonyles consiste à faire réagir un composé aromatique avec le chromehexacarbonyle dans divers solvants tels que des hydrocarbures et/ou des éthers ou au sein d'un excès du composé aromatique de départ. Cette méthode convient tout particulièrement bien à la synthèse d'arènechrometricarbonyles comportant des substituants électrodonneurs.

Plus spécifiquement encore la présente invention a pour objet un procédé de préparation d'acides aromatiques et de leurs esters répondant à la formule générale :

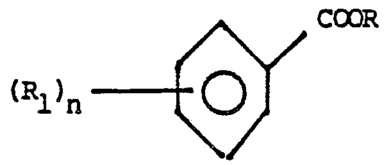

(I)

par carbonylation de composés halogénoarènechrometricarbonyles de formule générale :

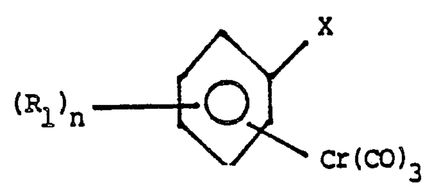

(II)

dans lesquelles :

R représente un atome d'hydrogène ou un radical alkyle, cycloalyle ou aryalkyle ayant de 1 à 20 atomes de carbone,

$R_1$ représente un radical alkyle ayant de 1 à 20 atomes de carbone ; un radical alkoxy de 1 à 20 atomes de carbone ; un atome de fluor ; un reste alkyle mono- ou polyhalogéné ; un groupe formyle ; un groupe carbonyloxyalkyle ; un groupe amino tertiaire, un groupe hydroxyle ; un reste acyle ; un reste acylamine,

n représente un nombre entier de 1 à 3 et de préférence de 1 à 2 ;

X représente un atome de chlore ou de brome.

Comme exemples spécifiques de radicaux R on peut citer à titre non limitatif des radicaux méthyle, éthyle, n-propyle, isopropyle, n-butyle, isobutyle, sec-butyle, n-pentyle, éthyl-2 hexyle, dodécyle, octadécyle, cyclohexyle, méthylcyclohexyle, benzyle, phényl-2 éthyle. De préférence R représente un radical alkyle inférieur, c'est-à-dire un radical alkyle ayant de 1 à 4 atomes de carbone.

Parmi les radicaux $R_1$ on peut citer des radicaux alkyles tels que ceux énumérés pour R ; des radicaux alkoxy tels que méthoxy, éthoxy, n-propoxy, isopropoxy, n-butyloxy, éthyl-2 hexyloxy, dodécyloxy, hexadécyloxy ; des radicaux halogéno alkyles tels que trifluorométhyle, difluorochlorométhyle, perfluoroéthyle ; des groupes carbonyloxyalkyle dans lesquels le reste alkyle comporte de 1 à 4 atomes de carbone tels que les groupes carbonylométhyle, carbonyloxyéthyle, carbonyloxyisopropyle ; des restes amino tertiaires de formule

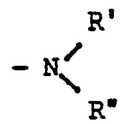

dans laquelle R' et R'' identiques ou différents représentent des radicaux alkyles ayant de 1 à 20 atomes de carbones tels que ceux cités pour R, des radicaux arylalkyles, des radicaux phényles ; des restes acyle et acylamino de formules

$R'''-CO-$ et 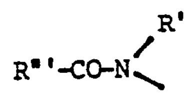

dans lesquelles R''' représente des radicaux alkyles, cycloalkyles ou arylalkyles tels que ceux cités pour R et des radicaux aryles (phényle, toluyle, naphtyles). Les radicaux acétyle, propionyle, benzoyle, méthylbenzoyles, acétylméthylamino, éthylbenzoylamino représentent des exemples spécifiques de radicaux acyles et acylamino. De préférence lorsque X représente un atome de brome $R_1$ représente un reste électrodonneur.

Comme exemples d'halogénoarènechrometricarbonyles on peut citer plus particulièrement :

le chlorobenzènechrometricarbonyle,
le chlorotoluènechrometricarbonyle,
le bromotoluènechrometricarbonyle,
le fluorochlorobenzènechrometricarbonyle,
le méthyl-1 fluoro-4 chloro-2 benzènechrometricarbonyle,
le chloro-1 trifluorométhyl-4 benzènechrometricarbonyle,
le chloro-1 tertiobutyl-4 benzènechrometricarbonyle,
le chloro-1 méthoxy-4 benzènechrometricarbonyle,
le bromo-1 méthoxy-4 benzènechrometricarbonyle,
le chloro-1 méthoxy-2 benzènechrometricarbonyle,
le chloro-1 hydroxy-4 benzènechrometricarbonyle,
le chloro-1 hydroxy-2 benzènechrometricarbonyle,
le bromo-1 diéthylamino-4 benzènechrometricarbonyle,
le chloro-1 méthoxycarbonyl-3 benzènechrometricarbonyle,
le bromo-2 méthoxycarbonyle-3 benzènechrometricarbonyle,
le chloro-1 acétyl-4 benzènechrometricarbonyle.

Le procédé selon l'invention convient particulièrement bien à la préparation d'acides tels que les acides benzoïque, toluiques, p-méthoxybenzoïque, p-fluorobenzoïque, p-trifluorométhylbenzoïque, p-hydroxybenzoïque, p-diéthylaminobenzoïque et leurs esters.

Sans que l'invention soit en rien limitée à un mécanisme particulier, la réaction de carbonylation peut être représentée par le schéma suivant :

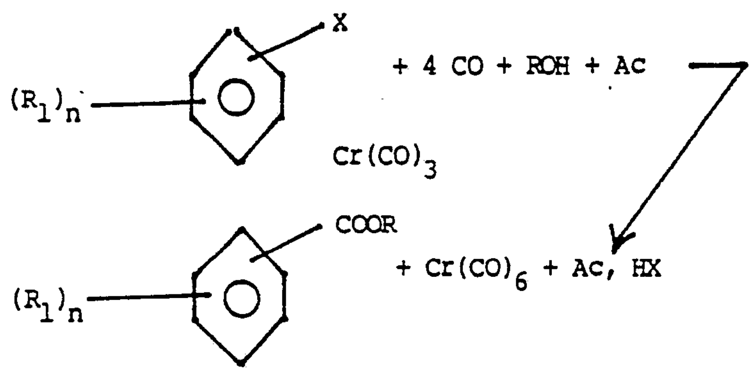


(1)

dans lequel ROH représente l'eau ou un alcool et Ac un accepteur d'hydracide.

Pour la mise en œuvre du procédé selon l'invention on peut faire appel à tout accepteur d'hydracide soluble dans le milieu ou miscible avec les composants du milieu réactionnel ; on peut utiliser des hydroxydes alcalins ou alcalino-terreux (soude, potasse, chaux) ; des oxydes alcalino-terreux (CaO) ; des carbonates et bicarbonates alcalins ou alcalino-terreux (carbonate et bicarbonate de sodium) ; des alcoolates alcalins (méthylate de sodium, éthylate de potassium, isopropylate de sodium, t-butylate de sodium) ; des carboxylates alcalins ou alcalino-terreux dérivés d'acides carboxyliques faibles (acides carboxyliques ayant un pK dans l'eau à 25 °C supérieur à 4) tels que les acétates, propionates, butyrates, isobutyrates, benzoates, phénylacétates de sodium ou de potassium ; des amines tertiaires et des bases hétérocycliques azotées tertiaires telles que la triméthylamine, la triéthylamine, la méthyltriéthylamine, la pyridine, les picolines, les N-alkylpipéridines (N-méthyl- ou N-éthylpipéridines par exemple). Les amines tertiaires et les bases azotées hétérocycliques tertiaires constituent une classe préférée d'accepteurs d'hydracides.

La quantité d'accepteur d'hydracide est au moins voisine de la quantité stœchiométrique nécessaire à la neutralisation de l'hydracide formé au cours de la réaction, c'est-à-dire voisine d'au moins 1 mole par mole d'halogénoarènechrometricarbonyle. De préférence on met en œuvre au moins 1,1 mole d'accepteur d'hydracide par mole d'halogénoarènechrometricarbonyle. Il n'y a pas de limite supérieure critique de la quantité d'accepteur d'hydracide et dans le cas des bases azotées tertiaires ces dernières peuvent constituer le milieu réactionnel. Pour les autres accepteurs d'hydracides il n'est en général pas nécessaire de recourir à plus de 1,5 mole d'accepteur par mole d'halogénoarènechrometricarbonyle ; une quantité comprise dans un intervalle de 1 à 1,25 est en général satisfaisante.

La nature du produit obtenu dépend de la nature du co-réactif. Lorsque ce réactif est l'eau on obtient un acide aromatique et lorsque ce réactif est un alcool la réaction d'alkoxycarbonylation conduit à la formation d'un ester. Dans ce dernier cas on fait appel à des alcools de formule générale :

$$R—OH \qquad (III)$$

dans laquelle R a la signification donnée pour la formule (I). On peut ainsi avoir recours à des alcanols tels que le méthanol, l'éthanol, le propanol-1, l'isopropanol, le t-butanol, l'isobutanol, l'éthyl-2 hexanol-1, l'alcool laurique, le cyclohexanol, l'alcool benzylique. De préférence on utilise les alcanols inférieurs et plus spécialement le méthanol et l'éthanol. Les alcools peuvent être anhydres ou contenir de l'eau ; la réaction conduit alors à la formation concomittante d'un acide et de son ester.

La quantité de co-réactif n'est pas critique. Il est cependant préférable pour assurer une transformation aussi complète que possible de l'halogénoarènechrometricarbonyle de mettre en œuvre une quantité d'eau et/ou d'alcool au moins voisine de la quantité stœchiométrique, c'est-à-dire voisine d'au moins 1 mole par mole d'halogénoarènechrometricarbonyle. Il n'y a pas de limite supérieure critique et l'eau et/ou les alcools peuvent constituer le milieu réactionnel en totalité ou en partie.

Les complexes palladium/phosphines utilisés comme catalyseurs dans le procédé selon la présente invention sont ceux habituellement mis en œuvre dans les réactions d'hydroxycarbonylation ou d'alkoxycarbonylation des halogénures aromatiques (cf. M. HIDAI, loc. cit.). On peut donc faire appel indifféremment à des complexes du palladium zéro ou à des complexes du palladium divalent ; ces derniers sont en général préférés en raison de leur stabilité à l'air qui les rend plus faciles à manipuler. Plus spécifiquement on peut faire appel à des complexes du palladium de formules générales :

$$Pd(CO)\ P[(R_2)_3]_3 \qquad (IV)$$

$$Pd_3(CO)_3[P(R_2)_3]_m \qquad (V)$$

$$Pd\ [P(R_2)_3]_4 \qquad (VI)$$

$$PdZ_2[P(R_2)_3]_2 \qquad (VII)$$

dans lesquelles :

$R_2$ représente un radical hydrocarboné aliphatique ou cycloaliphatique saturé ou aromatique comportant de 1 à 20 atomes de carbone éventuellement substitué par un ou plusieurs groupes fonctionnels hydrophyles inertes vis-à-vis de la réaction de carbonylation.

Z représente un atome d'halogène ou un reste acyloxy de formule $R_3$—COO— dans laquelle $R_3$ représente un radical alkyle, cycloalkyle, arylalkyle ayant de 1 à 20 atomes de carbone tel que ceux cités plus haut pour R ou aryle ;

m est 3 ou 4.

Dans les formules (IV) à (VII) les radicaux $R_2$, qui peuvent être identiques ou différents, représentent plus spécialement des radicaux méthyle, éthyle, propyle, isopropyle, n-butyle, sec-butyle, n-octyle, cyclohexyle, méthylcyclohexyle, phényle, toluyles, éthylphényles, xylyles, naphtyles, benzyle, phényl-2

éthyle. Comme exemple de groupe fonctionnel hydrophyle on peut citer le reste hydroxysulfonyle et les restes sulfonates alcalins ou alcalino-terreux. De préférence un au moins des radicaux $R_2$ représente un radical électrodonneur et plus préférentiellement encore un radical aryle. Z représente de préférence un atome de chlore ou de brome ou un radical acyloxyaliphatique saturé inférieur (acétoxy, propionyloxy, butyryloxy) ou un reste acyloxyaromatique (benzoyloxy, toluyloxy). Comme exemple de complexes palladium/phosphine on peut citer à titre non limitatif le tris-(méthyldiphénylphosphino) palladiummonocarbonyle, le tris-(triphénylphosphino) palladiummonocarbonyle, le tris-(éthylditolylphosphino) palladiummonocarbonyle, le tris-(éthylditolylphosphino) palladium, le tétrakis-(tritolylphosphino) palladium, le dichloro-bis-(triphénylphosphino) palladium, le dichloro-bis-(tritolylphosphino) palladium, le diisopropionyloxy-bis-(triphénylphosphino) palladium, les complexes dichloro- ou diacétoxy-bis(phosphino) palladium dans lesquels la phosphine est la tris-(hydroxysulfonylphényl) phosphine ou ses sels de sodium ou de potassium. On fait appel de préférence aux dihalogéno-bis-(triarylphosphino) palladium et aux diacyloxy-bis-(triarylphosphino) palladium.

Le procédé selon l'invention est conduit en présence d'un solvant des divers composés participant à la réaction, inerte dans les conditions de la réaction. Comme cela a été indiqué plus haut un premier groupe de solvants utilisables à cet effet est constitué par les bases tertiaires azotées utilisées comme accepteur d'hydracide, l'eau et/ou les alcools. On peut également faire appel à d'autres composés organiques tels que les hydrocarbures aliphatiques saturés (hexane par exemple), cycloaliphatiques saturés (cyclohexane, méthylcyclohexane), aromatiques (benzène, toluène, xylènes, éthylbenzène), des hydrocarbures aromatiques halogénés (chlorobenzène, chlorotoluènes), des éthers aliphatiques, des éthers cycliques (tétrahydrofuranne), aromatiques (anisole, chloroanisoles, bromoanisoles), des lactames (N-méthylpyrrolidone). D'une manière générale on fait appel à des composés organiques dont le caractère électrodonneur est le plus faible possible. D'un point de vue pratique il s'avère particulièrement avantageux d'utiliser comme solvant ou co-solvant le dérivé halogénoaromatique dont dérive l'halogénoarènechrometricarbonyle lorsqu'il est liquide dans les conditions normales de pression et de température. On prépare alors ce dernier par réaction du chromehexacarbonyle avec un excès de composé halogénoaromatique ; la solution d'halogénoarènechrometricarbonyle ainsi obtenue est directement utilisée dans la réaction de carbonylation.

La concentration des réactifs dans le milieu réactionnel n'est pas critique et peut varier dans de larges limites.

La quantité de catalyseur au palladium exprimée en nombre d'atome-gramme de palladium par mole d'halogénoarènechrometricarbonyle peut varier dans de larges limites. En général elle peut être comprise dans un intervalle allant de $1 \times 10^{-5}$ à 0,5 atome-gramme de palladium par mole d'halogénoarènechrometricarbonyles ; des quantités allant de $1 \times 10^{-4}$ à $1 \times 10^{-1}$ at.g de palladium par mole d'halogénoarènechrometricarbonyles conviennent bien.

La carbonylation des halogénoarènechrometricarbonyles est de préférence conduite en présence d'une phosphine libre qui est avantageusement la même que celle liée au palladium du catalyseur. La quantité de phosphine exprimée en at.g de phosphore par at.g de palladium peut varier dans de larges limites en fonction de la concentration en palladium dans le milieu ; le rapport phosphine libre/palladium est de préférence d'autant plus élevé que la concentration en palladium dans le milieu est plus faible. On a recours de façon générale à une quantité de phosphine telle que le nombre d'at.g de phosphore par at.g de palladium soit compris dans un intervalle de 0,5 à 1 000 et de préférence de 2 à 100.

On pourrait, sans sortir du cadre de la présente invention, générer « in situ » les espèces catalytiques nécessaires au déroulement de la réaction en chargeant dans le milieu réactionnel un complexe du palladium(+ 2) ou un sel d'acides carboxyliques du palladium(+ 2) (acétate palladeux, propionate palladeux) et la quantité adéquate de phosphine libre.

La réaction peut être conduite dans un large intervalle de températures. En général une température comprise dans un intervalle de 50 à 250 °C convient bien. De préférence on opère à une température de 80 à 200 °C.

Le procédé selon l'invention peut être mis en œuvre sous une pression d'oxyde de carbone égale à la pression atmosphérique ou sous pression plus élevée. De préférence la pression d'oxyde de carbone est inférieure à 150 bars et plus préférentiellement encore à 80 bars. Des pressions comprises entre 1 et 50 bars conviennent bien.

D'un point de vue pratique le procédé est réalisé simplement par passage d'un courant d'oxyde de carbone dans une solution aqueuse ou organique de l'halogénoarènechrometricarbonyle et des autres composés nécessaires au déroulement de la réaction ou par action d'une pression d'oxyde de carbone sur ladite solution, dans un récipient agité résistant à la pression. En fin de réaction les divers constituants du milieu réactionnel sont séparés par les techniques usuelles. La chromehexacarbonyle formé au cours de la réaction peut être isolé et recyclé à la préparation de l'halogénoarènechrometricarbonyle.

Les exemples suivants illustrent l'invention et montrent comment elle peut être mise en pratique. Les halogénoarènechrometricarbonyles utilisés dans les exemples ont été préparés par chauffage à reflux d'un composé halogénoaromatique avec le chromehexacarbonyle dans un mélange de tétrahydrofuranne et d'oxyde de butyle.

Exemple 1

Dans un autoclave en acier inoxydable de 100 ml équipé d'un système de chauffage et d'un agitateur magnétique, on charge :

2 m.moles de chlorobenzènechrometricarbonyle
0,04 m.mole de $PdCl_2[P(C_6H_6)_3]_2$
0,3 m.mole de triphénylphosphine
20 ml de méthanol
0,3 ml (2,16 m.mole) de triéthylamine.

L'autoclave est purgé par un courant de CO puis fermé. On charge ensuite de l'oxyde de carbone jusqu'à une pression de 25 bars, puis porte le mélange des réactifs à 180 °C sous agitation et maintient ces conditions pendant 20 heures. Après quoi le contenu de l'autoclave est refroidi à 20 °C puis dégazé.

On prélève une partie aliquote de masse réactionnelle sur laquelle on identifie les produits formés par spectrographie de masse et RMN du proton. Le benzoate de méthyle est dosé par chromatographie en phase gazeuse à ionisation de flamme. On constate qu'il s'est formé 1,6 m.mole de benzoate de méthyle, ce qui représente 80 % du chlorobenzènetricarbonyle chargé initialement (rendement réel).

Exemple 2

On opère comme à l'exemple 1 mais en chargeant 2,7 m.moles de chlorobenzènechromecarbonyle ; 0,003 m.mole de $PdCl_2(P[C_6H_5]_3)_2$ ; 0,45 ml de triéthylamine (3,24 m.moles) et 0,035 m.mole de triphénylphosphine. On dose en fin de réaction 0,95 m.mole de benzoate de méthyle (soit un rendement réel de 35 %) et 1,75 m.mole de chlorobenzène qui peut être recyclé à la synthèse du chlorobenzènechrometricarbonyle de sorte que le rendement par rapport au chlorobenzène présent dans le chlorobenzènechrometricarbonyle transformé (rendement théorique) s'élève à 100 %.

Exemple 3

On a opéré comme à l'exemple 1 mais en abaissant la température à 100 °C. On a obtenu un rendement réel en benzoate de méthyle de 67 %.

Exemple 4

On a répété l'exemple 1 mais en opérant sous une pression absolue d'oxyde de carbone de 1 bar. On a obtenu un rendement réel en benzoate de méthyle de 48 %.

Exemple 5

On a opéré comme à l'exemple 1 mais en arrêtant la réaction après 2 heures à 180 °C sous 25 bars de CO. On a obtenu un rendement réel en benzoate de méthyle de 80 %.

Exemple 6

On a opéré comme à l'exemple 1 mais après avoir remplacé la triéthylamine par l'acétate de sodium. On a obtenu un rendement réel de 68 %.

Exemple 7

On a opéré comme à l'exemple 1 mais en présence de 5 ml de chlorobenzène. On a obtenu un rendement réel de 26 %.

Exemples 8 à 11

On a opéré comme à l'exemple 1 à 130 °C et après avoir remplacé le chlorobenzènechrometricarbonyle par divers halogénoarènechrometricarbonyles. Les résultats obtenus sont consignés dans le tableau suivant :

(Voir Tableau page 7)

| EXEMPLES | HALOGENOARENECHROME TRICARBONYLE | RR % en benzoate de méthyle substitué |
|---|---|---|
| 8 | chloro-1 méthyl-4 BCTC (*) | 70 |
| 9 | chloro-1 trifluorométhyl-4 BCTC | 80 |
| 10 | chloro-1 méthoxy-4 BCTC | 55 |
| 11 | bromo-1 diméthylamino-4 BCTC | 50 |

(*) BCTC signifie « benzènechrometricarbonyle ».

## Revendications

1. Procédé de préparation d'acides carboxyliques aromatiques et/ou de leurs esters par carbonylation de composés chloro- ou bromoaromatiques en présence d'eau et/ou d'un alcanol, d'un accepteur d'hydracide, d'un complexe palladium/phosphine et éventuellement d'une phosphine caractérisé en ce que l'on soumet à la carbonylation des chloro- ou bromoarènechromecarbonyles.

2. Procédé selon la revendication 1, caractérisé en ce que l'on prépare des esters de formule générale :

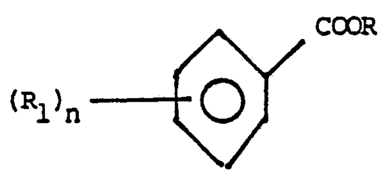

(I)

par carbonylation de composés halogénoarènechrometricarbonyles de formule générale :

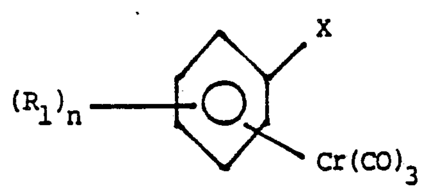

(II)

dans lesquelles :

R représente un atome d'hydrogène ou un radical alkyle, cycloalyle ou arylalkyle ayant de 1 à 20 atomes de carbone,

$R_1$ représente un radical alkyle ayant de 1 à 20 atomes de carbone ; un radical alkoxy de 1 à 20 atomes de carbone ; un atome de fluor ; un reste alkyle mono- ou polyhalogéné ; un groupe formyle ; un groupe carbonyloxyalkyle ; un groupe amino tertiaire, un groupe hydroxyle ; un reste acyle ; un reste acylamine,

n représente un nombre entier de 1 à 3 et de préférence de 1 à 2 ;

X représente un atome de chlore ou de brome.

3. Procédé selon la revendication 3, caractérisé en ce que dans les formules (I) et (II) R représente un radical alkyle inférieur.

4. Procédé selon l'une quelconque des revendications 1 à 3, caractérisé en ce que l'alcool utilisé comme co-réactif répond à la formule générale :

$$R—OH \qquad (III)$$

dans laquelle R a la signification donnée à la revendication 2.

5. Procédé selon l'une quelconque des revendications 1 à 4, caractérisé en ce que l'accepteur d'hydracide est pris dans le groupe formé par les hydroxydes alcalins ou alcalino-terreux, les carbonates ou bicarbonates alcalins ou alcalino-terreux, les alcoolates alcalins, les carboxylates alcalins ou alcalino-terreux, les amines tertiaires, les bases hétérocycliques azotées tertiaires.

6. Procédé selon l'une quelconque des revendications 1 à 5, caractérisé en ce que la quantité d'accepteur d'hydracide est d'au moins environ 1 mole par mole d'halogénoarènechrometricarbonyle.

7. Procédé selon l'une quelconque des revendications 1 à 6, caractérisé en ce que la quantité d'eau et/ou d'alcool est d'au moins environ 1 mole par mole d'halogénoarènechrometricarbonyle.

8. Procédé selon l'une quelconque des revendications 1 à 7, caractérisé en ce qu'on utilise comme catalyseur des complexes palladium/phosphine pris dans le groupe formé par les composés de formules :

$$Pd(CO)\ P[(R_2)_3]_3 \qquad (IV)$$

$$Pd_3(CO)_3[P(R_2)_3]_m \qquad (V)$$

$$Pd[P(R_2)_3]_4 \qquad (VI)$$

$$PdZ_2[P(R_2)_3]_2 \qquad (VII)$$

dans lesquelles :

$R_2$ représente un radical hydrocarboné aliphatique ou cycloaliphatique saturé ou aromatique comportant de 1 à 20 atomes de carbone ;

Z représente un atome d'halogène ou un reste acyloxy de formule $R_3$—COO— dans laquelle $R_3$ représente un radical alkyle, cycloalkyle, arylalkyle ayant de 1 à 20 atomes de carbone tel que ceux cités plus haut pour R ou aryle ;

m est 3 ou 4.

9. Procédé selon la revendication 8, caractérisé en ce que l'on utilise comme catalyseurs des composés de formules (IV) à (VII) dans lesquelles l'un au moins des radicaux $R_2$ représente un radical aryle.

10. Procédé selon la revendication 9, caractérisé en ce que l'on utilise le dichloro-bis(triphénylphosphino) palladium comme catalyseur.

11. Procédé selon l'une quelconque des reventications 1 à 10, caractérisé en ce que la quantité de catalyseur exprimée en atome-gramme de palladium par mole d'halogénoarènechrometricarbonyle est comprise dans un intervalle de $1 \times 10^{-5}$ à 0,5 atome-gramme par mole d'halogénoarènechrometricarbonyle.

12. Procédé selon l'une quelconque des revendications 1 à 11, caractérisé en ce que l'on opère en présence d'une quantité de phosphine libre exprimée en at.g de phosphore par atome-gramme de palladium comprise dans un intervalle de 0,5 à 1 000.

13. Procédé selon la revendication 12, caractérisé en ce que la phosphine utilisée est la même que celle présente dans le couple phosphine/palladium.

14. Procédé selon l'une quelconque des revendications 1 à 13, caractérisé en ce que l'on conduit la réaction dans l'eau ou dans un solvant organique inerte.

15. Procédé selon la revendication 14, caractérisé en ce que l'on utilise comme solvant organique la base azotée tertiaire utilisée comme accepteur d'hydracide.

16. Procédé selon la revendication 14, caractérisé en ce que l'on utilise comme solvant organique un excès de l'alcool participant à la réaction.

17. Procédé selon la revendication 14, caractérisé en ce que l'on utilise comme solvant organique un hydrocarbure aliphatique saturé, un hydrocarbure cycloaliphatique saturé, un hydrocarbure aromatique, un hydrocarbure aromatique halogéné, un éther, un lactame.

18. Procédé selon la revendication 14, caractérisé en ce que l'on utilise comme solvant organique le composé halogénoaromatique dont dérive l'halogénoarènechrometricarbonyle.

19. Procédé selon l'une quelconque des revendications 1 à 18, caractérisé en ce que la température de la réaction est comprise dans un intervalle de 50 à 250 °C.

20. Procédé selon l'une quelconque des revendications 1 à 19, caractérisé en ce que la pression absolue d'oxyde de carbone est comprise dans un intervalle de 1 à 150 bars.

**Claims**

1. Process for preparing aromatic carboxylic acids and/or their esters by the carbonylation of chloro- or bromoaromatic compounds in the presence of water and/or an alcohol, an acceptor for hydracid, a palladium/phosphine complex and optionally a phosphine, characterized in that chloro- or bromoarenechromium carbonyls are subjected to carbonylation.

2. Process according to claim 1, characterized in that esters of general formula :

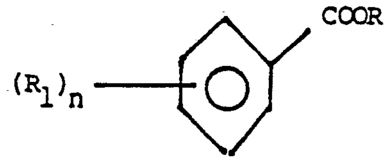

(I)

are prepared by the carbonylation of haloarenechromium tricarbonyl compounds of general formula :

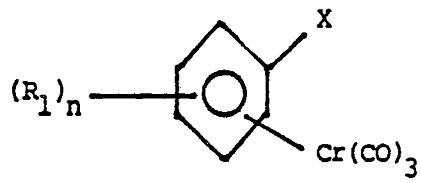

(II)

in which :

R denotes a hydrogen atom or an alkyl, cycloalkyl or arylalkyl radical having from 1 to 20 carbon atoms,

$R_1$ denotes an alkyl radical having from 1 to 20 carbon atoms ; an alkoxy radical of 1 to 20 carbon atoms ; a fluorine atom, a mono- or polyhalogenated alkyl residue ; a formyl group ; a carbonyloxy-alkyl group ; a tertiary amino group ; a hydroxyl group ; an acyl residue ; or an acylamine residue,

n denotes an integer from 1 to 3, and preferably from 1 to 2, and

X denotes a chlorine or bromine atom.

3. Process according to claim 2, characterized in that, in the formulae (I) and (II), R denotes a lower alkyl radical.

4. Process according to any one of claims 1 to 3, characterized in that the alcohol used as a coreactant corresponds to the general formula :

$$R—OH \quad (III)$$

in which R has the meaning given in claim 2.

5. Process according to any one of claims 1 to 4, characterized in that the acceptor for hydracid is chosen from the group composed of alkali metal or alkaline earth metal hydroxides, alkali metal or alkaline earth metal carbonates or bicarbonates, alkali metal alcoholates, alkali metal or alkaline earth metal carboxylates, tertiary amines and tertiary nitrogenous heterocyclic bases.

6. Process according to any one of claims 1 to 5, characterized in that the quantity of acceptor for hydracid is at least about 1 mole per mole of haloarenechromium tricarbonyl.

7. Process according to any one of claims 1 to 6, characterized in that the quantity of water and/or alcohol is at least about 1 mole per mole of haloarenechromium tricarbonyl.

8. Process according to any one of claims 1 to 7, characterized in that there are used as catalyst palladium/phosphine complexes chosen from the group composed of the compounds of formulae :

$$Pd(CO)P[(R_2)_3]_3 \quad (IV)$$

$$Pd_3(CO)_3[P(R_2)_3]_m \quad (V)$$

$$Pd[P(R_2)_3]_4 \quad (VI)$$

$$PdZ_2[P(R_2)_3]_2 \quad (VII)$$

in which :

$R_2$ denotes an aliphatic or saturated cycloaliphatic or aromatic hydrocarbon radical containing from 1 to 20 carbon atoms :

Z denotes a halogen atom or an acyloxy residue of formula $R_3$—COO-, in which $R_3$ denotes an alkyl, cycloalkyl or arylalkyl radical having from 1 to 20 carbon atoms, such as those mentioned above for R, or an aryl radical ; and m is 3 or 4.

9. Process according to claim 8, characterized in that compounds of formulae (IV) to (VII) in which at least one of the radicals $R_2$ denotes an aryl radical are used as catalysts.

10. Process according to claim 9, characterized in that dichlorobis (triphenylphosphino) palladium is used as catalyst.

11. Process according to any one of claims 1 to 10, characterized in that the quantity of catalyst, expressed as gram-atom of palladium per mole of haloarenechromium tricarbonyl, is within a range from $1 \times 10^{-5}$ to 0.5 gram-atom per mole of haloarenechromium tricarbonyl.

12. Process according to any one of claims 1 to 11, characterized in that the reaction is performed in the presence of a quantity of free phosphine, expressed in g-at. of phosphorus per gram-atom of palladium, within a range from 0.5 to 1 000.

13. Process according to claim 12, characterized in that the phosphine used is the same as that present in the phosphine/palladium system.

14. Process according to any one of claims 1 to 13, characterized in that the reaction is performed in water or in an inert organic solvent.

15. Process according to claim 14, characterized in that the tertiary nitrogenous base used as acceptor for hydracid is used as organic solvent.

16. Process according to claim 14, characterized in that an excess of the alcohol participating in the reaction is used as organic solvent.

17. Process according to claim 14, characterized in that a saturated aliphatic hydrocarbon, a saturated cycloaliphatic hydrocarbon, an aromatic hydrocarbon, a halogenated aromatic hydrocarbon, an ether or a lactam is used as organic solvent.

18. Process according to claim 14, characterized in that the haloaromatic compound from which the haloarenechromium tricarbonyl is derived is used as organic solvent.

19. Process according to any one of claims 1 to 18, characterized in that the reaction temperature is within a range from 50 to 250 °C.

20. Process according to any one of claims 1 to 19, characterized in that the absolute pressure of carbon monoxide is within a range from 1 to 150 bars.

**Patentansprüche**

1. Verfahren zur Herstellung von aromatischen Carbonsäuren und/oder ihrer Ester durch Carbonylierung von Chlor- oder Bromaromaten in Gegenwart von Wasser und/oder eines Alkanols, eines Wasserstoffsäureakzeptors, eines Palladium/Phosphin- Komplexes und gegebenenfalls eines Phosphins, dadurch gekennzeichnet, daß man Chlor- oder Bromaromaten-Chrom-Carbonyle der Carbonylierung unterwirft.

2. Verfahren nach Anspruch 1, dadurch gekennzeichnet, daß man Ester der allgemeinen Formel :

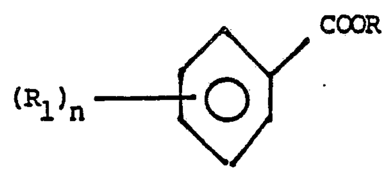

(I)

durch Carbonylierung von Halogenaromaten-Chrom-Tricarbonylen der allgemeinen Formel :

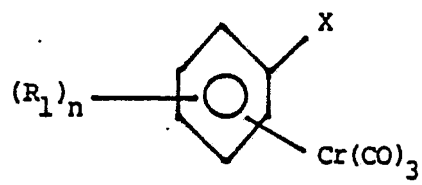

(II)

in denen :

R ein Wasserstoffatom oder ein Alkyl-, Cycloalkyl- oder Arylalkyl-Rest mit 1 bis 20 Kohlenstoffatomen bedeutet,

$R_1$ ein Alkylradikal mit 1 bis 20 Kohlenstoffatomen ; ein Alkoxyradikal mit 1 bis 20 Kohlenstoffatomen ; ein Fluoratom ; ein mono- oder polyhalogenierter Alkylrest ; eine Formylgruppe ; eine Carbonyloxyalkyl-Gruppe ; eine tertiäre Aminogruppe, eine Hydroxylgruppe ; eine Acylrest ; einen Acylaminrest bedeutet,

n eine ganze Zahl zwischen 1 und 3, vorzugsweise 1 oder 2 bedeutet ;

X ein Chlor- oder Bromatom bedeutet.

3. Verfahren nach Anspruch 3, dadurch gekennzeichnet, daß in den Formeln (I) und (II) R einen niederen Alkylrest bedeutet.

4. Verfahren nach einem der Ansprüche 1 bis 3, dadurch gekennzeichnet, daß der als Co-Reaktant eingesetzte Alkohol der allgemeinen Formel :

$$R—OH \quad (III)$$

entspricht, in der R die Bedeutung hat, die in Anspruch 2 angegeben ist.

5. Verfahren nach einem der Ansprüche 1 bis 4, dadurch gekennzeichnet, daß Wasserstoffsäureakzeptor der Gruppe entnommen ist, die durch die Alkali- oder Erdalkalihydroxide, Alkali- oder Erdalkalicarbonate oder bicarbonate, Alkalialkoholate, Alkali- oder Erdalkalicarboxylate, tertiäre Amine, tertiäre heterocyklische Stickstoffbasen gebildet wird.

6. Verfahren nach einem der Ansprüche 1 bis 5, dadurch gekennzeichnet, daß die Menge des Wasserstoffsäureakzeptors wenigstens ungefähr 1 Mol pro Mol des Halogenaromaten-Chrom-Tricarbonyls beträgt.

7. Verfahren nach einem der Ansprüche 1 bis 6, dadurch gekennzeichnet, daß die Menge des Wassers und/oder des Alkohols wenigstens ungefähr 1 Mol pro Mol des Halogenaromaten-Chrom-Tricarbonyls beträgt.

8. Verfahren nach einem der Ansprüche 1 bis 7, dadurch gekennzeichnet, daß man als Katalysator Palladium/Phosphinkomplexe benutzt, die der Gruppe entnommen sind, die durch die Verbindungen der Formeln :

$$Pd(CO)P[(R_2)_3]_3 \quad (IV)$$

$$Pd_3(CO)_3[P(R_2)_3]_m \quad (V)$$

$$Pd[P(R_2)_3]_4 \quad (VI)$$

$$PdZ_2[P(R_2)_3]_2 \quad (VII)$$

gebildet wird, in denen :

$R_2$ den Rest eines aliphatischen oder gesättigten cykloaliphatischen oder aromatischen Kohlenwasserstoffs mit 1 bis 20 Kohlenstoffatomen bedeutet ;

Z ein Halogenatom oder einen Acyloxyrest der Formel $R_3$—COO— bedeutet, in dem $R_3$ ein Alkyl-, Cykloalkyl-Arylalkyl-Radikal mit 1 bis 20 Kohlenstoffatomen bedeutet, wie es oben für R oder Aryl angegeben ist ;

m gleich 3 oder 4 ist.

9. Verfahren nach Anspruch 8, dadurch gekennzeichnet, daß man als Katalysatoren Verbindungen der Formel (IV) bis (VII) benutzt, in denen wenigstens einer der Reste $R_2$ ein Arylradikal bedeutet.

10. Verfahren nach Anspruch 9, dadurch gekennzeichnet, daß man Dichlor-bis (triphenylphosphino) palladium als Katalysator benutzt.

11. Verfahren nach einem der Ansprüche 1 bis 10, dadurch gekennzeichnet, daß die Menge des Katalysators ausgedrückt in Grammatomen an Palladium pro Mol Halogenaromat-Chrom-Tricarbonyl in einem Bereich von $1 \times 10^{-5}$ bis 0,5 Grammatom pro Mol Halogenaromat-Chrom-Tricarbonyl liegt.

12. Verfahren nach einem der Ansprüche 1 bis 11, dadurch gekennzeichnet, daß man in Gegenwart einer Menge von freiem Phosphin arbeitet, die, ausgedrückt in Grammatomen an Phosphor pro Grammatom Palladium, im Bereich zwischen 0,5 und 1 000 liegt.

13. Verfahren nach Anspruch 12, dadurch gekennzeichnet, daß das eingesetzte Phosphin das gleiche ist, das im Phosphin/Palladium-Paar vorhanden ist.

14. Verfahren nach einem der Ansprüche 1 bis 13, dadurch gekennzeichnet, daß man die Reaktion in Wasser oder einem inerten organischen Lösungsmittel durchführt.

15. Verfahren nach Anspruch 14, dadurch gekennzeichnet, daß man als organisches Lösungsmittel die tertiäre Stickstoffbase einsetzt, die man als Wasserstoffsäureakzeptor benutzt.

16. Verfahren nach Anspruch 14, dadurch gekennzeichnet, daß man als organisches Lösungsmittel einen Überschuß des Alkohols verwendet, der an der Reaktion teilnimmt.

17. Verfahren nach Anspruch 14, dadurch gekennzeichnet, daß man als organisches Lösesmittel einen gesättigten aliphatischen Kohlenwasserstoff, einen gesättigten cykloaliphatischen Kohlenwasserstoff, einen aromatischen Kohlenwasserstoff, einen halogenierten aromatischen Kohlenwasserstoff, einen Ether, ein Laktam benutzt.

18. Verfahren nach Anspruch 14, dadurch gekennzeichnet, daß man als organisches Lösemittel den Halogenaromaten benutzt, von dem sich das Halogenaromaten-Chrom-Tricarbonyl ableitet.

19. Verfahren nach einem der Ansprüche 1 bis 18, dadurch gekennzeichnet, daß die Reaktionstemperatur im Bereich zwischen 50 und 250 °C liegt.

20. Verfahren nach einem der Ansprüche 1 bis 19, dadurch gekennzeichnet, daß der Absolutdruck an Kohlenoxyd im Bereich zwischen 1 und 150 bar liegt.